# EUROPEAN PATENT APPLICATION

(11) **EP 3 263 146 A1**
(43) Date of publication of application: **03.01.2018**
(21) Application number: 16755629.9
(22) Date of filing: 25.02.2016
(51) Int. Cl.: A61L 31/00

(54) **METHOD FOR MANUFACTURING MEDICAL MATERIAL, MEDICAL MATERIAL, AND ANTI-ADHESION MATERIAL**

(30) Priority: 27.02.2015 JP 2015038008
(71) Applicant: Dainichiseika Color & Chemicals Mfg. Co., Ltd., Chuo-ku Tokyo 103-8383 (JP)
(72) Inventor: ISONO, Yasuyuki, Tokyo 103-8383 (JP); NOISHIKI, Yasuharu, Yokohama-shi Kanagawa 236-0005 (JP)
(74) Representative: Blodig, Wolfgang
(86) International application number: PCT/JP2016/055655
(87) International publication number: WO 2016/136885

(57) **Abstract**

There is provided a process for producing a medical material that retains the properties which are inherent in a polyanionic polysaccharide being a raw material, that has a high level of safety because there is no need to use a chemical crosslinking agent, and that has moderate strength and flexibility. The present invention is a process for producing a medical material, the process including a step of dispersing a powder or a granular product of a first polyanionic polysaccharide, the first polyanionic polysaccharide water-insolubilized with a treatment liquid containing a first acid anhydride, in an aqueous solution of a water-soluble salt of a second polyanionic polysaccharide, thereby obtaining a dispersion liquid, a step of drying the dispersion liquid obtained, thereby obtaining a dried film, and a step of water-insolubilizing the dried film obtained with a treatment liquid containing a second acid anhydride, thereby obtaining the medical material.

## Description

### Technical Field

The present invention relates to a process for producing a medical material, a medical material, and an anti-adhesion material.

### Background Art

It is known that polyanionic polysaccharides such as hyaluronic acid and alginic acid exhibit moderate viscosity, adhesiveness, moisture retention, and biocompatibility. Therefore, these polyanionic polysaccharides and salts thereof are widely used as a raw material for medical materials, food materials, cosmetic materials, and the like.

Hyaluronic acid among others is excellent in its characteristic physical properties such as water retention and has a high level of safety and biocompatibility, so that it is used in various applications such as foods, cosmetics, and pharmaceutical products. For example, in the medical field, hyaluronic acid is used in raw materials for joint lubricants and anti-adhesion materials. However, sodium hyaluronate to be a raw material has a high water-solubility and therefore needs to be subjected to certain insolubilization treatment depending on the application.

Various studies on the method for water-insolubilizing sodium hyaluronate through crosslinking reaction making use of a carboxy group have been made so far. For example, Patent Literature 1 describes a method for producing a water-insoluble derivative of a polyanionic polysaccharide such as hyaluronic acid and carboxymethyl cellulose through crosslinking reaction using a carbodiimide.

In addition, Patent Literatures 2 and 3 describe a method for water-insolubilizing a polyanionic polysaccharide such as hyaluronic acid and carboxyalkyl cellulose by forming ionic bonds using a polyvalent cation. Further, Patent Literature 4 describes a method for obtaining a water-insolubilized film by subjecting carboxymethyl cellulose to ion exchange using a metal salt.

Patent Literature 5 describes a method for water-insolubilizing sodium hyaluronate by cooling a sodium hyaluronate aqueous solution to -20°C under an acidic condition to form intramolecular crosslinks. In addition, Patent Literature 6 describes acetylation through reaction of hyaluronic acid in a powder form with acetic anhydride in the presence of concentrated sulfuric acid. Further, Patent Literature 7 describes a method for producing hyaluronic acid gel using an acidic liquid containing an alcohol.

### Citation List

### Patent Literature

Patent Literature 1: National Publication of International Patent Application No. 2003-518167
Patent Literature 2: Japanese Patent Application Laid-Open No. 5-124968
Patent Literature 3: Japanese Patent Application Laid-Open No. 2008-13510
Patent Literature 4: Japanese Patent Application Laid-Open No. 6-128395
Patent Literature 5: Japanese Patent Application Laid-Open No. 2003-252905
Patent Literature 6: Japanese Patent Application Laid-Open No. 8-53501
Patent Literature 7: Japanese Patent Application Laid-Open No. 5-58881

### Summary of Invention

### Technical Problem

However, a crosslinking agent is used in the method described in Patent Literature 1, and therefore it is often difficult to apply the method for applications where the safety should be taken into consideration because products are applied to human bodies, such as pharmaceutical products. In addition, the extent of water-insolubility for the obtained films and the like are not described at all in Patent Literatures 2 to 4.

Further, in the method described in Patent Literature 5, the pH of the sodium hyaluronate aqueous solution needs to be adjusted at about 1.2, and the viscosity increases outstandingly, so that handling at the time of shaping and the like is difficult. In addition, freeze drying is conducted for long hours, so that there has also been a problem in terms of cost of electric power required for cooling. Further, when the sodium hyaluronate aqueous solution is stored under an acidic condition, the viscosity increases rapidly to make the shaping difficult, so that the application may be limited. It is to be noted that in Patent Literature 5, the intramolecular crosslinked structure is identified, but the extent of insolubilization is not referred to.

In addition, the extent of water-insolubility for the obtained acetylated product of hyaluronic acid is not described at all in Patent Literature 6. Further, the hyaluronic acid gel obtained by the method described in Patent Literature 7 contains a large amount of water and therefore is hard even to lift. Thus, it is difficult to insolubilize the shaped body while keeping the shape of the shaped body.

The present invention has been completed in consideration of the problems of the conventional techniques, and a subject matter of the present invention is to provide a process for producing a medical material: that retains the properties which are inherent in a polyanionic polysaccharide being a raw material; that has a high level of safety because there is no need to use a chemical crosslinking agent; and that has moderate strength and flexibility. In addition, another subject matter of the present invention is to provide a medical material and an anti-adhesion material produced through the above-described method.

### Solution to Problem

That is, according to the present invention, a process for producing a medical material described below is provided.
[1] A process for producing a medical material, the process including: a step of dispersing a powder or a granular product of a first polyanionic polysaccharide, the first polyanionic polysaccharide water-insolubilized with a treatment liquid containing a first acid anhydride, in an aqueous solution of a water-soluble salt of a second polyanionic polysaccharide, thereby obtaining a dispersion liquid; a step of drying the dispersion liquid obtained, thereby obtaining a dried film; and a step of water-insolubilizing the dried film obtained with a treatment liquid containing a second acid anhydride, thereby obtaining the medical material.
[2] The process for producing a medical material according to [1], wherein the first polyanionic polysaccharide and the second polyanionic polysaccharide are each at least one selected from the group consisting of hyaluronic acid, carboxymethyl cellulose, and alginic acid.
[3] The process for producing a medical material according to [1] or [2], wherein the first acid anhydride and the second acid anhydride are each at least any one of acetic anhydride and propionic anhydride.
   In addition, according to the present invention, a medical material described below is provided.
[4] A medical material produced by the production process according to any one of [1] to [3].
   Further, according to the present invention, an anti-adhesion material described below is provided.
[5] An anti-adhesion material containing a polyhydric alcohol or a polyhydric alcohol aqueous solution retained in the medical material according to [4].

### Advantageous Effects of Invention

According to the process for producing a medical material of the present invention, a medical material that retains the properties which are inherent in a polyanionic polysaccharide being a raw material, that has a high level of safety because there is no need to use a chemical crosslinking agent, and that has moderate strength and flexibility can be produced easily.

### Description of Embodiments

Hereinafter, embodiments according to the present invention will be described; however, the present invention is not limited to the embodiments below.

### (Medical Material and Process for Producing the Medical Material)

The method for producing a medical material according to the present invention includes: a step (dispersion step) of dispersing a powder or a granular product of a first polyanionic polysaccharide, the first polyanionic polysaccharide water-insolubilized with a treatment liquid containing a first acid anhydride, in an aqueous solution of a water-soluble salt of a second polyanionic polysaccharide, thereby obtaining a dispersion liquid; a step (drying step) of drying the dispersion liquid, thereby obtaining a dried film; and a step (water-insolubilization step) of water-insolubilizing the dried film with a treatment liquid containing a second acid anhydride. Hereinafter, the details will be described.

The powder or the granular product of the first polyanionic polysaccharide for use in the dispersion step can be obtained, for example, by water-insolubilizing a powder of a water-soluble salt of the first polyanionic polysaccharide with the treatment liquid containing the first acid anhydride. In addition, the powder or the granular product of the first polyanionic polysaccharide can also be obtained by water-insolubilizing a sponge-like raw material-shaped body made of a raw material containing a water-soluble salt of the first polyanionic polysaccharide with the treatment liquid containing the first acid anhydride, and then pulverizing the water-insolubilized product by using a cutter mill or the like, or other methods. It is to be noted that the sponge-like raw material-shaped body can be produced, for example, by pouring an aqueous solution of the water-soluble salt of the first polyanionic polysaccharide into an appropriate container, and then drying or freeze-drying the aqueous solution.

The first polyanionic polysaccharide is a polysaccharide having one or more negatively charged anionic groups such as a carboxy group and a sulfonic acid group in the molecular structure thereof. In addition, the water-soluble salt of the first polyanionic polysaccharide is a salt formed from at least a part of the anionic groups in the first polyanionic polysaccharide. It is to be noted that the anionic group in the first polyanionic polysaccharide may be the one that is introduced in a molecule of a polysaccharide.

Specific examples of the first polyanionic polysaccharide include carboxyalkyl cellulose such as carboxymethyl cellulose and carboxyethyl cellulose, carboxymethyl starch, carboxymethylamylose, chondroitin sulfate (including chondroitin-4-sulfate and chondroitin-6-sulfate), hyaluronic acid, heparin, heparin sulfate, heparan sulfate, alginic acid, pectin, carrageenan, dermatan sulfate, and dermatan-6-sulfate. These first polyanionic polysaccharides can be used singly or in a combination of two or more.

Examples of the water-soluble salt of the first polyanionic polysaccharide include inorganic salts, ammonium salts, and organic amine salts. Specific examples of the inorganic salts include: alkali metal salts such as sodium salts and potassium salts; alkali earth metal salts such as calcium salts; and metal salts such as zinc salts and iron salts.

The treatment liquid which is used for water-insolubilizing the powder of the water-soluble salt of the first polyanionic polysaccharide contains a first acid anhydride. Specific examples of the acid anhydride include acetic anhydride, propionic anhydride, succinic anhydride, butyric anhydride, phthalic anhydride, and maleic anhydride. Among them, acetic anhydride and propionic anhydride are preferable. These acid anhydrides can be used singly or in a combination of two or more.

It is preferable that the treatment liquid further contain at least one medium of water and a water-soluble organic solvent, and it is also preferable that the first acid anhydride be dissolved or dispersed in the medium. By using the treatment liquid in which the first acid anhydride is dissolved or dispersed in the medium, the powder of the water-soluble salt of the first polyanionic polysaccharide can be water-insolubilized sufficiently and immediately.

Specific examples of the water-soluble organic solvent include methanol, ethanol, propanol, dimethyl sulfoxide (DMSO), acetonitrile, and tetrahydrofuran. Among them, methanol, ethanol, and dimethylsulfoxide are preferable. These water-soluble organic solvents can be used singly or in a combination of two or more.

The concentration of the first acid anhydride in the treatment liquid is usually 0.1 to 50% by mass and is preferably 5 to 30% by mass. When the concentration of the first acid anhydride is less than 0.1% by mass, the extent of water-insolubilization is insufficient, or there is a tendency that the water-insolubilization takes a long time. On the other hand, when the concentration of the first acid anhydride exceeds 50% by mass, there is a tendency that the effects hit the ceiling.

It is preferable that the treatment liquid contain water as a medium from the viewpoint of water-insolubilizing the powder of the water-soluble salt of the first polyanionic polysaccharide further sufficiently and immediately. The content of water in the treatment liquid is preferably 0.01 to 50% by mass, more preferably 5 to 20% by mass. When the content of water in the treatment liquid is less than 0.01% by mass, the water-insolubilization may be insufficient for the solvents other than methanol. In addition, when the content of water in the treatment liquid exceeds 50% by mass, the powder of the water-soluble salt of the first polyanionic polysaccharide may easily dissolve.

A dispersion liquid in which the powder or the granular product of the first polyanionic polysaccharide is dispersed in an aqueous solution can be obtained in such a way that the powder or the like of the first polyanionic polysaccharide and an aqueous solution of the water-soluble salt of the second polyanionic polysaccharide are mixed and then subjected to stirring or the like appropriately. As the second polyanionic polysaccharide, the same polyanionic polysaccharides as the first polyanionic polysaccharides can be used. The first polyanionic polysaccharide and the second polyanionic polysaccharide may be the same or different. It is to be noted that the dispersion liquid may further contain a radiopaque agent including a contrast agent such as barium sulfate.

In the drying step, the obtained dispersion liquid is dried to obtain a dried film. Subsequently, in the water-insolubilization step, the dried film obtained in the drying step is water-insolubilized with the treatment liquid containing the second acid anhydride to obtain the medical material. As the second acid anhydride, the same acid anhydrides with the first acid anhydrides can be used. The first acid anhydride and the second acid anhydride may be the same or different.

By treating the dried film with the treatment liquid containing the second acid anhydride in the water-insolubilization step, the dried film is water-insolubilized while maintaining the shape thereof. The method for treating the dried film with the treatment liquid is not particularly limited; however, the treatment is preferably conducted in such a way that the treatment liquid is brought into contact with the whole dried film and the treatment liquid penetrates inside the dried film. Specific examples of the treatment method include a method in which the dried film is immersed in the treatment liquid and a method in which the treatment liquid is applied or sprayed (atomized) onto the dried film.

The temperature during the water-insolubilization treatment is not particularly limited as long as the temperature does not exceed the boiling point of the treatment liquid. It is preferable to set the temperature during the water-insolubilization treatment at 0 to 80°C, more preferably 0 to 70°C, and particularly preferably room temperature (25°C) to 60°C from the viewpoint of suppressing the decomposition and denaturation of the polyanionic polysaccharide and from the viewpoint of suppressing the volatilization of the medium, byproducts, and the like. However, when the treatment is conducted under the condition in which the treatment liquid does not volatilize during the water-insolubilization treatment, for example, the treatment is conducted with a heat press or a heat roller, the medical material can be obtained in a shorter time without undergoing the decomposition and denaturation, and the like. For example, in the case where the water-insolubilization treatment is conducted with a heat press or a heat roller, it is preferable that the temperature during the water-insolubilization treatment be set at 50 to 90°C, and the treatment time be set for 30 minutes or shorter. After the water-insolubilization treatment is completed, the medical material according to the present invention can be obtained, if necessary, through washing or the like with water or a water-soluble organic solvent.

The reaction that is supposed to progress when the shaped body formed using a sodium salt of the polyanionic polysaccharide is treated with an alcoholic solution of acetic anhydride is shown below. It is to be noted that the supposed reaction can be one of the factors for water-insolubilization, but there is a possibility that the water-insolubilization is achieved by a combination with another factor for water-insolubilization or by a totally different factor. That is, the present invention is not limited at all by the supposed reaction described below.

R₁-COON^{a} + (CH₃CO)₂O + R₂-OH → R₁-COOH + CH₃COON^{a} + CH₃COOR₂ ··· (1)

In the reaction formula (1), R₁ represents the main chain of the polyanionic polysaccharide, and R₂ represents the main chain of the alcohol. Acetic anhydride, when cleaved in the presence of an alcohol, deprives sodium of the polyanionic polysaccharide and the carboxy groups change into an acid form from a sodium salt form. The change can be confirmed by measuring the Na content or by titration with an alkaline solution.

In the case where water exists in the reaction system, it is envisaged that the reaction represented by the following reaction formula (2) progresses in addition to and in parallel with the reaction represented by the reaction formula (1) and the carboxy groups change into an acid form from a sodium salt form.

R₁-COON^{a} + (CH₃CO)₂O + H₂O → R₁-COOH + CH₃COON^{a} + CH₃COOH ··· (2)

It is to be noted that all the anionic groups in a molecule may not necessarily be in the acid form in the obtained medical material.

It is extremely difficult to obtain a shaped body that is sufficiently water-insolubilized even when a shaped body or the like formed using the water-soluble salt of the polyanionic polysaccharide is immersed in an inorganic acid such as hydrochloric acid or an organic acid such as acetic acid. In addition, a water-insolubilized, shaped body cannot be obtained even when the acid anhydride in the treatment liquid is replaced with an acid that corresponds to the acid anhydride. From these facts, it is envisaged that the water-insolubilization is achieved by a different factor combined with the factor that the anionic groups in the polyanionic polysaccharide change into the acid form.

There is no need to use a chemical crosslinking agent during the production of the medical material according to the present invention, and therefore a structure of a functional group or the like which is derived from the chemical crosslinking agent is not incorporated into the molecule. Therefore, the medical material according to the present invention retains the properties which are inherent in the polyanionic polysaccharide being a raw material and has a high level of safety. In addition, the medical material according to the present invention produced in the manner as described above has moderate strength and flexibility. Accordingly, the medical material according to the present invention is suitable as an anti-adhesion material and the like. It is to be noted that in the case where the medical material according to the present invention is used as a constituent material for an anti-adhesion material, the thickness of the medical material is not particularly limited, but is preferably 20 to 200 µm, more preferably 60 to 120 µm.

The molecules of the polyanionic polysaccharide which constitutes the medical material according to the present invention are not substantially crosslinked. Further, a new covalent bond is not substantially formed in the polyanionic polysaccharide. However, it is inferred that physical bonds such as hydrogen bonds, hydrophilic bonds, and van der Walls force are formed between the molecules of the polyanionic polysaccharide. Formation of such physical bonds between the molecules of the polyanionic polysaccharide can be confirmed by measuring an infrared absorption spectrum.

The medical material according to the present invention is stably water-insoluble in a wide pH range from acidity to alkalinity. However, the medical material according to the present invention, when brought into contact with or immersed in an aqueous medium having a pH of 12 or higher, can be dissolved easily due to dissociation of the physical bonds between the molecules.

### (Anti-Adhesion Material)

The anti-adhesion material according to the present invention contains a polyhydric alcohol or a polyhydric alcohol aqueous solution retained in the medical material. Specific examples of the polyhydric alcohol include ethylene glycol, diethylene glycol, polyethylene glycol, methyl glycerol, polyoxyethylene glycoside, maltitol, mannitol, xylitol, sorbitol, reduced sugar syrup, dipropylene glycol, butylene glycol, valine, propylene glycol, glycerin (glycerol), polyglycerin, and fatty acid esters of glycerin. Among them, polyhydric alcohols which are used in the medical field and the food field, such as glycerin, xylitol, sorbitol, and low-molecular-weight polyethylene glycols, are used suitably. With respect to these suitably usable polyhydric alcohols, products sold on the market can be used as they are. With respect to glycerin, sorbitol, and the like, it is desirable to use products which conform to the Japanese pharmacopoeia. Glycerin is a material having a high level of safety to such an extent that can also be used as injections into a vein and therefore is greatly preferable.

Examples of the method for allowing the polyhydric alcohol or the polyhydric alcohol aqueous solution to be retained in the medical material include a method in which the medical material is immersed in the polyhydric alcohol or the polyhydric alcohol aqueous solution having a predetermined concentration. That is, by immersing the medical material in the polyhydric alcohol aqueous solution to replace the inside of the medical material with the polyhydric alcohol aqueous solution, the polyhydric alcohol aqueous solution in a desired concentration is retained, so that a desired anti-adhesion material according to the present invention can be obtained. It is to be noted that the thickness of the anti-adhesion material according to the present invention is not particularly limited, but is preferably 20 to 200 µm, more preferably 60 to 120 µm.

### Examples

Hereinafter, the present invention will be described specifically based on Examples; however, the present invention is not limited to the Examples. It is to be noted that the "part (s) " and "%" in Examples and Comparative Examples are on a mass basis unless otherwise noted.

### (Example 1)

In 100 mL of an 80% ethanol aqueous solution, 1.0 g of a powder of sodium hyaluronate (molecular weight of 800000 Da) was dispersed. The resultant dispersion liquid was heated to 50°C under stirring, and 20 mL of acetic anhydride were then added thereto to stir the resultant mixture for further 1 hour. A precipitate collected by centrifugal separation was washed with ethanol and water and was then dried and pulverized to obtain a hyaluronic acid powder. In 50 mL of a 1% sodium hyaluronate (molecular weight of 800000 Da) aqueous solution, 0.5 g of the obtained hyaluronic acid powder were dispersed, and the resultant dispersion liquid was poured into a stainless-steel tray of 12-cm length x 10-cm width and was then dried in a thermostatic chamber at 20°C to obtain a dried film. The dried film obtained was immersed in a treatment liquid (solution of 20% acetic anhydride/80% ethanol) and was then left to stand at 50°C for 1 hour to be water-insolubilized and thereby obtain a hyaluronic acid powder-hyaluronic acid composite film having a thickness of about 50 µm. The obtained hyaluronic powder-hyaluronic acid composite film had moderate strength and flexibility.

### (Example 2)

Into a stainless-steel tray of 12-cm length x 10-cm width, 50 mL of a 1% sodium hyaluronate (molecular weight of 800000 Da) aqueous solution were poured and then frozen in a freezer at -80°C. The frozen aqueous solution was subjected to vacuum freeze drying (degree of vacuum of -20 Pa, shelf temperature of 25°C) to obtain a sponge made of sodium hyaluronate. The obtained sponge was immersed in a treatment liquid (solution of 20% acetic anhydride/80% ethanol) and was then left to stand at 50°C for 1 hour to be water-insolubilized and thereby obtain a sponge made of hyaluronic acid. The obtained sponge was pulverized using a cutter mill to obtain a crushed product (granular product) of hyaluronic acid. In 50 mL of a 1% sodium hyaluronate (molecular weight of 800000 Da) aqueous solution, 0.5 g of the crushed product obtained were dispersed, and the resultant dispersion liquid was poured into a stainless-steel tray of 12-cm length x 10-cm width and was then dried in a thermostatic chamber at 20°C to obtain a dried film. The dried film obtained was immersed in a treatment liquid (solution of 20% acetic anhydride/80% ethanol) and was then left to stand at 50°C for 1 hour to be water-insolubilized and thereby obtain a hyaluronic acid-crushed product (granular product)-hyaluronic acid composite film having a thickness of about 60 µm. The hyaluronic acid-crushed product (granular product)-hyaluronic acid composite film obtained had moderate strength and flexibility.

### (Evaluation 1: Solubility Test)

The composite films produced in respective Examples were each cut into a 2-cm square and put into a container having a diameter of 3.5 cm and a depth of 1.5 cm, and 5 mL of a PBS buffer solution (pH of 6.8) were added thereto. The container was placed in a shaker the temperature of which was adjusted at 37°C and was then shaken at 10 to 20 rpm, and the changes of the state over time were visually observed. As a result, it was understood that the original shape of the film was retained even after 72 hours and the film was water-insolubilized for any of the composite films. In addition, the swelling ratio (swollen film/dried film (mass ratio)) after 72 hours was 2.4.

### (Comparative Example 1)

Into a stainless-steel tray of 12-cm length x 10-cm width, 50 mL of a 1% sodium hyaluronate (molecular weight of 800000 Da) aqueous solution were poured and was then dried in a thermostatic chamber at 20°C to obtain a dried film. The dried film obtained was immersed in a treatment liquid (solution of 20% acetic anhydride/80% ethanol) and was then left to stand at 50°C for 1 hour to be water-insolubilized and thereby obtain a water-insolubilized hyaluronic acid film having a thickness of about 50 µm.

### (Evaluation 2: Tensile Test)

A tensile test was conducted in accordance with JIS K 7311 (Testing methods for thermoplastic polyurethane elastomers) to measure the tensile strength of the composite film produced in Example 1. Firstly, the composite film sufficiently swollen with distilled water was punched with a dumbbell cutter to prepare a test piece. Subsequently, the breaking strength was measured using a single column type material testing instrument (trade name "STA-1150", manufactured by A&D Company, Ltd.) at a crosshead speed of 10 mm/sec to calculate the tensile strength of the composite film. The tensile strength of the composite film produced in Example 1 was 3 N/mm². In addition, the tensile strength of the water-insolubilized hyaluronic acid film produced in Comparative example 1, which was measured and calculated by the same procedure, was 1.5 N/mm².

### (Example 3)

The composite film produced in Example 1 was immersed in a 10% by volume glycerin aqueous solution. The composite film was then air-dried and thereafter sealed in a sterilization bag. Sterilization was conducted to the composite film together with the sterilization bag through irradiation with 25 kGy radiation rays to obtain an anti-adhesion film having a thickness of about 50 µm. A laparotomy was performed to a mature dog (beagle dog, female, 1.5 years old, weight of about 10 kg) after general anesthesia, and the epidermis of a ventral wall was peeled into a 3-cm square. The anti-adhesion film was placed so as to cover the peeled portion, and an abdominal closure was then performed. Two weeks later, a laparotomy was performed to the same dog after general anesthesia to find that adhesion had not occurred. In addition, the anti-adhesion film placed (implanted) in the body of the dog was found to have disappeared two weeks after the implantation. As the reason, it is inferred that the carboxy groups in hyaluronic acid, which constitutes the anti-adhesion film, were gradually neutralized by sodium ions or the like in the living body to cause the hyaluronic acid to change into a water-soluble hyaluronic acid salt, so that the hyaluronic acid dissolved to be absorbed into the living body. In contrast, with respect to a dog to whom an abdominal closure was performed without placing the anti-adhesion film, it was observed that adhesion had occurred between the peeled portion and the bowel.

### Industrial Applicability

The medical material according to the present invention is useful as a material for constituting an anti-adhesion material.

## Claims

1. A process for producing a medical material, the process comprising:
a step of dispersing a powder or a granular product of a first polyanionic polysaccharide, the first polyanionic polysaccharide water-insolubilized with a treatment liquid comprising a first acid anhydride, in an aqueous solution of a water-soluble salt of a second polyanionic polysaccharide, thereby obtaining a dispersion liquid;
a step of drying the dispersion liquid obtained, thereby obtaining a dried film; and
a step of water-insolubilizing the dried film obtained with a treatment liquid comprising a second acid anhydride, thereby obtaining the medical material.

2. The process for producing a medical material according to claim 1, wherein the first polyanionic polysaccharide and the second polyanionic polysaccharide are each at least one selected from the group consisting of hyaluronic acid, carboxymethyl cellulose, and alginic acid.

3. The process for producing a medical material according to claim 1 or 2, wherein the first acid anhydride and the second acid anhydride are each at least any one of acetic anhydride and propionic anhydride.

4. A medical material produced by the production process according to any one of claims 1 to 3.

5. An anti-adhesion material comprising a polyhydric alcohol or a polyhydric alcohol aqueous solution retained in the medical material according to claim 4.
